# EUROPEAN PATENT SPECIFICATION

(11) **EP 3 723 601 B1**
(45) Date of publication and mention of the grant of the patent: **06.03.2024**
(21) Application number: 18829784.0
(22) Date of filing: 14.12.2018
(51) Int. Cl.: A61B 5/087, A61B 5/113

(54) **SCREENING TOOL FOR PATIENTS PULMONARY CONDITIONS**
SCREENING-TOOL FÜR LUNGENERKRANKUNGEN VON PATIENTEN
OUTIL DE CRIBLAGE POUR DES PATIENTS SOUFFRANT D'AFFECTIONS PULMONAIRES

(30) Priority: 14.12.2017 EP 17207403
(43) Date of publication of application: 21.10.2020
(73) Proprietor: Fluidda Respi NV, 2550 Kontich (BE)
(72) Inventor: DE BACKER, Jan, 2880 Bornem (BE)
(74) Representative: De Clercq & Partners
(86) International application number: PCT/EP2018/084985
(87) International publication number: WO 2019/115779

(56) References cited:
- EP-A1- 2 435 118
- EP-B1- 2 435 118
- US-A1- 2011 060 215
- US-A1- 2015 265 187
- US-A1- 2016 030 689
- US-A1- 2017 119 255
- DE BACKER ET AL: "Computational fluid dynamics can detect changes in airway resistance in asthmatics after acute bronchodilation", JOURNAL OF BIOMECHANICS, PERGAMON PRESS, NEW YORK, NY, US, vol. 41, no. 1, 19 December 2007 (2007-12-19), pages 106-113, XP022394750, ISSN: 0021-9290
- DE BACKER ET AL: "Functional imaging using computational fluid dynamics to predict treatment success of mandibular advancement devices in sleep-disordered breathing", JOURNAL OF BIOMECHANICS, PERGAMON PRESS, NEW YORK, NY, US, vol. 40, no. 16, 1 January 2007 (2007-01-01), pages 3708-3714, XP022356299, ISSN: 0021-9290, DOI: 10.1016/J.JBIOMECH.2007.06.022 cited in the application
- DE BACKER J W ET AL: "Novel imaging techniques using computer methods for the evaluation of the upper airway in patients with sleep-disordered breathing: A comprehensive review", SLEEP MEDICINE REVIEWS, W.B. SAUNDERS, AMSTERDAM, NL, vol. 12, no. 6, 1 December 2008 (2008-12-01), pages 437-447, XP025654430, ISSN: 1087-0792, DOI: 10.1016/J.SMRV.2008.07.009 [retrieved on 2008-10-15] cited in the application
- ANDRIY MYRONENKO ET AL: "Point-Set Registration: Coherent Point Drift", ARXIV.ORG, CORNELL UNIVERSITY LIBRARY, 201 OLIN LIBRARY CORNELL UNIVERSITY ITHACA, NY 14853, 16 May 2009 (2009-05-16), XP080324657, DOI: 10.1109/TPAMI.2010.46 cited in the application

## Description

### TECHNICAL FIELD

The present invention is in the field of screening techniques for pulmonary conditions. In particular, the present invention provides systems and methods for screening a subject for the presence of pulmonary conditions.

### BACKGROUND

Spirometry is a commonly applied test used to assess breathing patterns and lung function, which may help determine both the presence and severity of lung diseases. It can, however, be argued that spirometry, and in particular the Forced Vital Capacity (FVC), indirectly leads to the observed high mortality in Pulmonary Fibrosis patients. FVC is one of the outcome parameters of spirometry and often the primary endpoint in clinical trials for new drugs in pulmonary fibrosis. To obtain an FVC the patient is asked to forcefully exhale into a machine that records how much air the patient can possibly exhale after taking the deepest breath possible. So FVC essentially provides the dynamic difference between total lung capacity (TLC) and residual volume (RV).

Lung Fibrosis is a respiratory disease characterized by scaring of the lung tissue, often without a known cause, resulting in a restrictive condition. For the same pleural pressures the affected parts of the lung expand less. It is commonly assumed that a reduction in FVC indicates smaller lung volumes and hence can be a good descriptor of the disease. The latter, however, is arguable not necessarily the case.

While it is true that eventually the lungs and consequently the FVC will become smaller, at the early stages of the disease the healthy areas of the lung commonly compensate for the affected parts as the disease progresses, thereby maintaining a normal lung function (i.e. normal FVC) despite declining lung health. Consequently, spirometry may serve as an indicator of how healthy the lungs are whereas for screening purposes it may be more interesting to assess how diseased (i.e. affected) they are instead.

For instance, clinical trials performed on patients suffering from Idiopathic pulmonary fibrosis (IPF) showed that even though the conventional lung function tests indicated that everything is normal, the disease has already significantly progressed. Late diagnosis of any disease is associated with poorer survival outcome. To improve patient care for subjects suffering from pulmonary conditions such as lung fibrosis an early detection of the condition is necessary.

Accordingly, several methods are known for assessing the lung's function and serve as screening tools to help determine potential pulmonary conditions. However, these are known to be imprecise or only cover specific diseases or conditions, are cumbersome and may even be painful for affected patients, require following tedious procedures and/or are available to only a small sub-group of patients. Hence, it is an aim of the invention to overcome the problems of the art. In particular, there is a need for improved systems, devices and/or methods that can better assist the screening for pulmonary conditions.

EP 2 435 118 A1 describes methods for determining ventilation parameters based on calculation of airflow dynamics through airway and lobar models of a subjects derived from medical imaging studies. De Backer et al "Computational fluid dynamics can detect changes in airway resistance in asthmatics after acute bronchodilation", Journal of Biomechanics, (2007), vol. 41, no. 1, pages 106-113, describes methods of computation fluid dynamics based on medical images of a patient to detect changes in airway resistance in asthmatics based on medical imaging data.

De Backer et al "Functional imaging using computational fluid dynamics to predict treatment success of mandibular advancement devices in sleep-disordered breathing", Journal of Biomechanics, (2007), vol. 40, no. 16, pages 3708-3714, describes methods of computation fluid dynamics based on medical images of a patient to determines effects of a mandibular advancement device on upper airway resistance in sleep-disorder patients.

De Backer J.M. et al "Novel imaging techniques using computer methods for the evaluation of the upper airway in patients with sleep-disordered breathing: A comprehensive review", Sleep Medicine Reviews, M.B. Saunders, (2008) vol. 12, no. 6, pages, 437-447 describes methods of computation fluid dynamics based on medical images of a patient to evaluate upper airway breathing in sleep-disorder patients.

Andriy Myronenko et al: "Point-Set Registration: Coherent Point Drift", Cornell University Library, 201 Olin Library Cornell University Ithaca, NY 14853, (2009), describes a probabilistic method for assigning correspondences between two sets of points and recovering a transformation that maps one point set to the other.

US 2011/060215 A1 describes using an ultra wide-band radar system having at least one transmitting and receiving antenna for applying ultra wide-band radio signals to a target area of a subject's anatomy in order to determine associated respiratory parameters.

US 2016/030689 A1 describes using chest sensor on a subject to monitor a local motion of the chest to monitor artificial ventilation and functional volume changes of the chest. It describes a ventilation model for a mechanical ventilation machine, and use of sensors in an animal trial for determining parameters such as tidal motion index, maximal inflation rate, maximal expiratory rate, and symmetric (left vs right) ventilation during artificial ventilation.

US 2017/0119225 A1 describes a multi-sensor cardio-respiratory device including acoustic sensors provided as a belt-type configuration to be worn at the upper abdomen.

US 2015/265187 A1 discloses systems and methods for non-contact monitoring of spatio-temporal mechanics comprising motion patterns of respiratory muscles, lungs and diaphragm, for improving respiratory mechanics of a subject.

### SUMMARY

The devices and methods according to the present disclosure solve the aforementioned problems. Accordingly, provided herein are systems and methods for screening techniques for pulmonary conditions.

Provided is a system in accordance with claim 1 comprising a sensor unit configured for registering an expansion of a thorax of a subject, the system being configured for determining from data outputted by the sensor unit a local expansion of the thorax and an internal airflow distribution, IAD, of the lungs of the subject, wherein
- the sensor unit sensor unit comprises at least one optical sensor unit and
- the IAD of the lungs of the subject comprises an IAD to lower lung lobes and an IAD to upper lung lobes, and
the at least one optical sensor unit comprises a scanner and/or a camera, configured to capture optical images of the upper thorax and the lower thorax of the subject..

The data outputted by the sensor unit comprises data corresponding to local upper and local lower thorax expansions of the subject.

The system may determine an IAD to the upper lung lobes and IAD to the lower lung lobes by querying a model or database created using a reference dataset of lobar upper and lower IADs and thoracic expansion patterns of healthy subjects and subjects with a pulmonary disorder.

The reference dataset of lADs and thoracic expansion patterns may be determined from medical images obtained by medical imaging of healthy subjects and subjects with a pulmonary disorder at total lung capacity and functional residual capacity.

Data outputted by the sensor unit may correspond to local upper and local lower thorax expansions of the subject, wherein a positional upper boundary to the upper thorax is defined by the 3rd intercostal space, and a positional lower boundary to the lower thorax is defined by the xiphoid process.

The internal airflow distribution of the lungs may comprise (information as to) an internal airflow to lower lung lobes and an internal airflow to upper lung lobes.

The internal airflow distribution of the lungs may comprise (information as to) an internal airflow to left lung lobes and internal airflow to right lung lobes.

The internal airflow distribution of the lungs may comprise (information as to) internal airflow to each and every lung lobe.

The sensor unit may comprise at least one mechanical sensor configured for skin-dismountable attachment to a thoracic region of the subject and/or at least one optical sensor unit.

The mechanical sensor may be configured for detection of strain, linear acceleration, rotation, vibration, or sound.

The sensor unit may comprise at least two mechanical sensors one configured for attachment to the upper thorax and one configured for attachment to the lower thorax of the subject.

The mechanical sensor may be a strain gauge and/or a piezo electric sensor, optionally wherein the sensor unit comprises a strain gauge and piezo electric sensor.

The at least one optical sensor unit may comprise a scanner and/or a camera.

The sensor unit may comprise at least two (e.g. 2, 3, 4, 5, 6, 7, 8, 9 or more) mechanical sensors, and optionally each mechanical sensor is configured for attachment to a different position of the thoracic region of the subject.

The system may be configured for determining a time-resolved internal airflow distribution of the lungs during a breathing cycle.

The system may be configured for determining an integrated internal airflow distribution of the lungs during exhalation and during inhalation.

The system may be further configured for determining the lower lobe volume and the upper lobe volume from a comparison between measurement data obtained during inhalation and measurement data obtained during exhalation.

The system may be further configured to determine, from the internal airflow distribution of the lungs of the subject, a status of the subject with respect to a pulmonary disorder.

The pulmonary disorder may be idiopathic pulmonary fibrosis, IPF, Asthma, COPD, CF, BOS, non-CF bronchiectasis, PH, BPD, A1AT, ILD, or any combination thereof.

The system identifies a normal status of subject when the IAD to the upper lung lobes is 40 to 45%, and when the IAD to the lower lung lobes is 55 to 60%.

Further provided is a computer-implemented method for determining internal airflow determining distribution, IAD, in lungs of a subject comprising the steps:
- receiving sensor data from a sensor unit comprising at least an optical sensor unit comprising a scanner and/or a camera, the sensor unit being configured for registering a local expansion of the thorax or a thoracic region of the subject, the sensor data corresponding to local upper and local lower thorax expansions of the subject;
- computing, from the sensor unit data, local expansion of the thorax and spatially and/or temporally resolved volume changes in the subject's lungs; and
- computing, from the volume changes, the IAD in the subject's lower lung lobes and upper lung lobes; wherein the airflow distribution is optionally temporally resolved.

The sensor unit may further comprise at least one mechanical sensor dismountably attached to skin of a thoracic region of a subject.

The method may further comprise the step of:
- computing from the airflow distribution in the subject's lungs, the IAD upper vs lower lobes ratio, the IAD left vs right lobes ratio, and/or the internal airflow to each and every lung lobe (ratio).

The method may further comprise the step of:
- determining from the airflow distribution in the subject's lungs, a status of the subject with respect to a pulmonary disorder.

Further provided is a use of a system according to one or more embodiments as described herein.

### DESCRIPTION OF THE FIGURES

The following description of the figures of specific embodiments of the invention is only given by way of example and is not intended to limit the present explanation, its application or use. In the drawings, identical reference numerals refer to the same or similar parts and features.
**FIG. 1** shows an exemplary flow-chart for performing the method according to one or more embodiments as described herein.
**FIG. 2** shows an exemplary flow-chart for preparing a model or database. Example 2 illustrates an exemplary implementation of said flow-chart.
**FIG. 3A** shows the thoracic expansion of a healthy subject; **FIG. 3B** shows the thoracic expansion of a subject affected with a pulmonary disorder. Example 3 details how the figures were obtained.
**FIG. 4** shows differences in internal airflow distribution (IAD) for upper lung (UL) lobes and lower lung (LL) lobes in healthy subjects and subject with idiopathic pulmonary fibrosis (IPF) in mild, moderate and severe conditions.

### DETAILED DESCRIPTION

As used below in this text, the singular forms "a", "an", "the" include both the singular and the plural, unless the context clearly indicates otherwise.

The terms "comprise", "comprises" as used below are synonymous with "including", "include" or "contain", "contains" and are inclusive or open and do not exclude additional unmentioned parts, elements or method steps. Where this description refers to a product or process which "comprises" specific features, parts or steps, this refers to the possibility that other features, parts or steps may also be present, but may also refer to embodiments which only contain the listed features, parts or steps.

The enumeration of numeric values by means of ranges of figures comprises all values and fractions in these ranges, as well as the cited end points.

The term "approximately" as used when referring to a measurable value, such as a parameter, an amount, a time period, and the like, is intended to include variations of +/- 10% or less, preferably +/-5% or less, more preferably +/-1% or less, and still more preferably +/-0.1% or less, of and from the specified value, in so far as the variations apply to the invention disclosed herein. It should be understood that the value to which the term "approximately" refers per se has also been disclosed.

All references cited in this description are hereby deemed to be incorporated in their entirety by way of reference.

Unless defined otherwise, all terms disclosed in the invention, including technical and scientific terms, have the meaning which a person skilled in the art usually gives them. For further guidance, definitions are included to further explain terms which are used in the description of the invention.

An aspect of the invention provides a system comprising a sensor unit configured for registering the expansion of the thorax of a subject, the system being configured for determining from data outputted by the sensor unit a local expansion of the thorax and (from the local expansion of the thorax) an internal airflow distribution (IAD) of the lungs of the subject. From the internal airflow distribution (IAD) of the lungs of the subject, a status of the presenting subject with respect to a pulmonary condition may be determined. The terms thorax and thoracic region have been used interchangeable herein. The thorax refers to the part of the body between the neck and abdomen. Measurements by the sensor unit are typically of the anterior of the subject with the subject lying in supine position (i.e. face and thorax facing upwards). Measurements by the sensor unit are typically taken on or in respect of the skin of the anterior thorax.

The presenting subject may be a healthy individual or a patient affected with one or more pulmonary conditions. The status of the presenting subject may be an indicator of the probability that a subject is affected with a pulmonary condition. For instance, a positive status or a probability may attributed to the presenting subject, which may serve as an indicator for determining whether the presenting subject is at risk for being affected by a pulmonary condition. The presenting subject with positive status may be referred for further assessment with other techniques.

The pulmonary conditions may be numerous; for example, it may be one or more of idiopathic pulmonary fibrosis (IPF), Asthma, Chronic Obstructive Pulmonary Disease (COPD), Cystic fibrosis (CF), Bronchiolitis Obliterans Syndrome (BOS), non-CF bronchiectasis, Pulmonary Hypertension (PH), Bronchopulmonary dysplasia (BPD), alpha-1 antitrypsin deficiency (A1AT), Interstitial lung disease (ILD), and others. Different pulmonary condition may affect different regions of the lungs at different rates. Moreover, a subject may be affected by combinations of pulmonary conditions, which can further contribute to affecting the breathing patterns and lung function of the subject. It is noted that alternating subject positions (e.g. supine, prone, tilted or lateral) may be appropriate when determining the risk of specific pulmonary conditions, such as airway obstructions or anatomical abnormalities.

In view of the above, the invention may allow for earlier and more accurate detection of potential risks factors suitable for determining whether a subject is at risk for being affected by a pulmonary condition. An earlier detection of a potential pulmonary condition allows for improved care and recovery rate for affected subjects. Moreover, the invention may allow for a faster detection when compared to (more advanced and invasive) diagnosing systems and methods, which may be less cumbersome and time-consuming for subjects and healthcare providers, and may be less costly for the healthcare system.

Furthermore, the present invention may also provide for a more accurate determining of the internal airflow distribution in lungs of a subject. This could for instance be used for evaluating the fitness of lungs in a specific subject; for example when examining athletes the regional ventilation can influence the ventilation to perfusion matching which is known to influence exercise tolerance; or alternatively, when evaluating a patient after long-related surgery the regional ventilation may serve as indicator of the rate of local recovery without masking the masking of compensating regions.

The present system and methods avoids the need to acquire medical images of subject in order to determine internal airflow distribution. Medical images refer to those taken within the body using radiological methods including HRCT scanner, X-ray radiography, ultrasound, computed tomography (CT), nuclear medicine including positron emission tomography (PET), and magnetic resonance imaging (MRI). These are understood to be different from images acquired using an optical sensor and light in the infrared, visible, and/or ultraviolet spectrum and relating to the bodily exterior. It is appreciated that medical images obtained from radiological methods may be used to create a database or train a model for transforming measurement data obtained by the sensor unit as discussed elsewhere herein.

The human respiratory system is situated in the thoracic cavity of the chest, or the thorax. During a breathing cycle the lungs are inflated and deflated to allow for sufficient gas exchange, which is caused by the thorax naturally expanding and contracting. Consequently, the local expansion of the thorax may be correlated with the distribution of the internal airflow (IAD) for each corresponding region of the lungs, which are commonly referred to as lobes. The lobes can be grouped into upper and lower lobes, wherein the upper lobes consist of right upper lobe, right middle lobe and left upper lobe, and the lower lobes consist of the right lower lobe and left lower lobe.

During a normal breathing cycle of healthy subjects (i.e. subjects not affected by a pulmonary condition) the internal airflow may be distributed amongst the lung lobes and corresponding lung zones. The total values (e.g. volume) may vary based on variables, such as gender, height, age, and physical fitness of the subject. However, the inventors observed that after reduction of the variables, the IAD was observed to be only dependent on the lung lobe or lung zone.

For a healthy subject (i.e. not affected by a pulmonary disorder) the internal airflow distribution (IAD) towards the right upper lung lobe (RUL) may comprise from 16.05% up to 17.19% of the total airflow, preferably may comprise approximately 17%; the IAD towards the left upper lung lobe (LUL) may comprise from 20.11% up to 21.48%, preferably may comprise approximately 21%; the IAD towards the right middle lung lobe (RML) may comprise from 6.05% up to 6.55%, preferably may comprise approximately 6%; the IAD towards the right lower lung lobe (RLL), may comprise from 25.73% to 27.39%, preferably may comprise 29%; the IAD towards the left lower lung lobe (LLL) may comprise from 27.85% to 29.61%, preferably may comprise 27% These listed ranges for healthy subjects may be referred to as the standard IAD values. A detailed overview of the standard IAD values observed for a healthy subject may be found in Example 1; in particular listed in Table 1.

In view of the above listed standard lAD ranges for every lung lobe an IAD based on one or more lung zones may be determined: firstly how the air is distributed between the upper lung lobes (e.g. RUL, LUL and RML) versus the lower lung lobes (e.g. RLL and LLL); and secondly how the air is distributed between the left lung lobes (e.g. LUL and LLL) versus the right lung lobes (e.g. RLL, RML and RLL).

Accordingly, for a healthy subject the IAD towards the upper lung lobes may comprise from 40 to 45%, 42.75% up to 44.61%, preferably may comprise approximately 43%; and the IAD towards the lower lung lobes may comprise from, 55 to 60%, 54.06% up to 56.48%, preferably may comprise approximately 55%. This specific distribution of airflow may be referred to as the IAD upper vs lower lung lobes ratio, or simply the upper vs lower IAD ratio. Thus when referring to the "normal" upper vs lower IAD ratio it may be understood that this refers to a range of (42.75-44.61):(54.06-56.48)%; preferably a ratio of approximately 43:55%.

Accordingly, for a healthy subject the IAD towards the left lung lobes may comprise from 46.26% up to 48.41%, preferably may comprise approximately 47%; and the IAD towards the lower lung lobes may comprise from 50.53% up to 52.70%, preferably may comprise approximately 52%. This specific distribution of airflow may be referred to as the IAD left vs right lung lobes ratio, or simply the left vs right IAD ratio. Thus when referring to the "normal" left vs right IAD ratio it may be understood that this refers to a range of (46.26-48.41):(50.53-52.70)%; preferably a ratio of 47:52%.

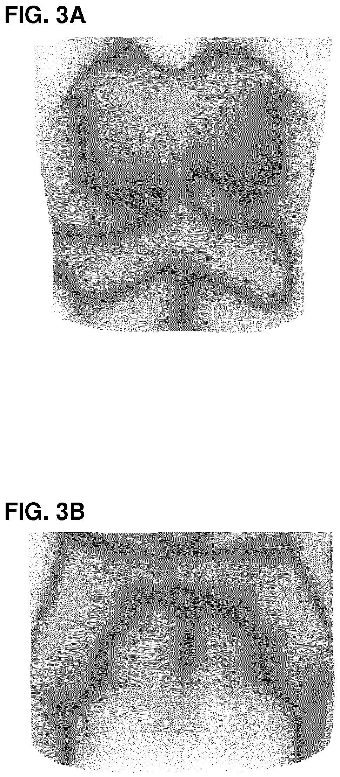

Subjects affected by one or more pulmonary conditions may have their IAD ratios altered slightly (e.g. ± 3-5%), moderately (e.g. ± 5-10%), significantly (e.g. ± 10-15%) or immensely (e.g. ± 15% and more). For example, for very mild lung fibrosis patients (with an FVC > 100%p) typically more air may be observed going towards the upper lobes (e.g. RUL, LUL, and RML) compared to the lower lobes (e.g. RLL and LLL). This could indicate that the healthy upper lobes are compensating for the affected lower lobes. Consequently, if during a (routine) screening performed on a particular subject the IAD ratio is observed to deviate from the normal IAD ratio, for instance a moderately altered IAD upper vs lower lung lobes ratio of 50:50 (%), the subject may be assigned a status, which may be indicative of a risk of a potential pulmonary condition. An exemplary embodiment may be found illustrated in Example 3; in particular figures 3A and 3B. The screenings may for instance be performed on subjects selected from a population considered at risk for developing lung fibrosis.

The measurement data outputted by the sensor unit may be extracted, converted and analysed. The data may comprise spatially and/or temporally resolved volume changes, which may or may not require the input of information from a user of the system. For instance, for determining spatially resolved volume changes the user may be requested to enter spatially related parameters, such as (relative) sensor position(s), patient parameters including thorax size, (estimated or measured) lung capacity, etc. Additionally or alternatively, the spatially related parameters may be measured and automatically entered by the sensor unit. Similarly, for determining temporally resolved airflow distribution in the lungs the user may be requested to enter temporally related parameters, such as the start of a breathing cycle, or they may be measured and automatically entered by the sensor unit.

In some embodiments the internal airflow distribution of the lungs comprises (information as to) an internal airflow to lower lung lobes and an internal airflow to upper lung lobes. The IAD upper vs lower lobes ratio may serve as an indicator for the presence of a pulmonary condition.

In some further embodiments the system determines an IAD of the upper vs lower lung lobes ratio. Preferably, the system determined IAD upper vs lower lung lobes ratio (%)may be at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 43:57 to 45:55; for example 44:56. As discussed above, any deviation from a standard IAD upper vs lower lobes ratio may serve as an indicator for the presence of a pulmonary condition.

In some embodiments the internal airflow distribution of the lungs comprises (information as to) an internal airflow to left lung lobes and internal airflow to right lung lobes. The IAD left vs right lung lobes ratio may also serve as an indicator for the presence of a pulmonary condition.

In some further embodiments the system determines an IAD left vs right lung lobes ratio. Preferably, the system determined IAD left vs right lung lobes ratio (%) may be at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 46:54 to 49:51; for example 47:53; for example 48:52;. As discussed above, any deviation from a standard IAD left vs right lobes ratio may serve as an indicator for the presence of a pulmonary condition.

In some further embodiments the internal airflow distribution of the lungs comprises (information as to) an internal airflow to lower lung lobes, an internal airflow to upper lung lobes, an internal airflow to left lung lobes and internal airflow to right lung lobes. Different pulmonary condition may affect different regions of the lungs, which may then be compensated by the complementary healthy regions (e.g. lower for upper and/or left for right, and vice versa). Consequently, determining internal airflow distribution for each lung region allow for more accurately determining a status of the subject with respect to a pulmonary disorder, and, by comparing data from each lung region, it may allow for at least generally localising the pulmonary disorder within a potentially affected lung region.

In some embodiments the internal airflow distribution of the lungs comprises (information as to) internal airflow to each and every lung lobe. The each and every lung lobe comprise (information as to) the internal airflow distribution of the right upper lobe (RUL), the internal airflow distribution of the left upper lobe (LUL), the internal airflow distribution of the right middle lobe (RML), the internal airflow distribution of the right lower lobe (RLL) and the internal airflow distribution of the left lower lobe (LLL). Determining the airflow to each lobe may provide for the highest accuracy and detect any compensation performed by any of the healthy or less affected lobe(s).

In some further embodiments the system determines an IAD lung lobes ratio for each and every lung lobe. Preferably, the system determined IAD lung lobe ratio (%) for the RUL lobe relative to the combined ratio of the other lung lobes (e.g. LUL, RML, RLL, and LLL) may comprise at least 0% to at most 40%; preferably at least 10% to at most 30%; more preferably 15% to at most 25%; most preferably 18% to 20%; for example 19%. Preferably, the system determined IAD lung lobe ratio (%) for the LUL lobe relative to the combined ratio of the other lung lobes (e.g. RUL, RML, RLL, and LLL) may comprise at least 0% to at most 40%; preferably at least 10% to at most 30%; more preferably 15% to at most 25%; most preferably 22 to 24%; for example 23%. Preferably, the system determined IAD lung lobe ratio (%) for the RML lobe relative to the combined ratio of the other lung lobes (e.g. RUL, LUL, RLL, and LLL) may comprise at least 0% to at most 20%; preferably at least 3% to at most 15%; more preferably 5% to at most 10%; most preferably 7% to 9%; for example 8%. Preferably, the system determined IAD lung lobe ratio (%) for the RLL lobe relative to the combined ratio of the other lung lobes (e.g. RUL, LUL, RML, and LLL) may comprise at least 0% to at most 40%; preferably at least 15% to at most 35%; more preferably 20% to at most 30%; most preferably 25% to 27%; for example 26%. Preferably, the system determined IAD lung lobe ratio (%) for the LLL lobe relative to the combined ratio of the other lung lobes (e.g. RUL, LUL, RML, and RLL) may comprise at least 0% to at most 40%; preferably at least 15% to at most 35%; more preferably 20% to at most 30%; most preferably 22% to 24%; for example 25%. As discussed above, any deviation from a standard lung lobes ratio may serve as an indicator for the presence of a pulmonary condition.

The local expansion of the thorax may also be correlated with the distribution of the ventilation in the lungs, in particular the air intake volume and/or capacity of each region of the lungs, in particular the lung lobes and/or lung zones. The lobar distribution may be determined with respect to the total lung capacity (TLC-LD), and/or with respect to the residual capacity (FRC-LD). A more detailed overview of the standard values for each lung lobe and lung zones for a healthy subject may be found in Example 1; in particular in Tables 2 and 3 respectively.

Similarly to the IAD, the lobar distribution may be determined from measurement data outputted by the sensor unit. The inventors observed that after reduction of variables, the lobar distribution was observed to be only dependent on the lung lobe or lung zone. Subjects affected by one or more pulmonary conditions may also have their lobar distribution ratios altered slightly (e.g. ± 3-5 %), moderately (e.g. ± 5-10 %), significantly (e.g. ± 10-15 %) or immensely (e.g. ± 15 % and more).

When measuring the total lung capacity (TLC-LD) for a healthy subject the right upper lung lobe (RUL) may comprise from 18.29% up to 19.25% of the total air volume, preferably may comprise approximately 18%; the left upper lung lobe (LUL) may comprise from 22.46% up to 23.59% of the total air volume, preferably may comprise approximately 23%; the right middle lung lobe (RML) may comprise from 7.97% up to 8.46% of the total air volume, preferably may comprise approximately 8%; the right lower lung lobe (RLL) may comprise from 25.29% up to 26.53% of the total air volume, preferably may comprise approximately 26%; the left lower lung lobe (LLL) may comprise from 22.91% to 24.06% of the total air volume, preferably may comprise approximately 23%. These listed ranges for healthy subjects may be referred to as the standard TLC-LD values. A more detailed overview of the standard TLC-LD values observed for a healthy subject may be found in Example 1; in particular listed in Table 2.

Accordingly, when measuring the TLC-LD for a healthy subject the upper lung lobes may comprise from 49.22% up to 50.78% of the total air volume, preferably comprise approximately 50%; whereas the lower lung lobes comprise from 48.54% up to 50.23% of the total air volume, preferably comprise approximately 50%. This specific distribution of airflow may be referred to as the TLC-LD upper vs lower lung lobes ratio, or simply the upper vs lower TLC-LD ratio. Accordingly, when referring to the "normal" upper vs lower TLC-LD ratio it may be understood that this refers to a range of (49.22-50.78):(48.54%-50.23)%; preferably is a ratio of approximately 50:50%.

Accordingly, when measuring the TLC-LD for a healthy subject the left lung lobes may comprise from 45.69% up to 47.30% of the total air volume, preferably comprise approximately 47%; whereas the right lung lobes comprise from 52.06% up to 53.71% of the total air volume, preferably comprise approximately 53%. This specific distribution of airflow may be referred to as the TLC-LD left vs right lung lobes ratio, or simply the left vs right TLC-LD ratio. Accordingly, when referring to the "normal" left vs right TLC-LD ratio it may be understood that this refers to a range of (45.69-47.30):(52.06-53.71)%; preferably is a ratio of 47:53%.

When measuring the functional residual capacity (FRC-LD) for a healthy subject the right upper lung lobe (RUL) may comprise from 20.26% up to 21.18% of the remaining air volume, preferably may comprise approximately 20%; the left upper lung lobe (LUL) may comprise from 24.55% up to 25.47% of the total air volume, preferably may comprise approximately 25%; the right middle lung lobe (RML) may comprise from 9.69% up to 9.69% of the total air volume, preferably may comprise approximately 10%; the right lower lung lobe (RLL) may comprise from 23.00% up to 23.92% of the total air volume, preferably may comprise approximately 23%; the left lower lung lobe (LLL) may comprise from 20.23% to 21.15% of the total air volume, preferably may comprise approximately 21%. These observed ranges for healthy subjects may be referred to as the standard FRC-LD values. A more detailed overview of the standard FRC-LD values observed for a healthy subject may be found in Example 1; in particular in Table 3.

Accordingly, when measuring the FRC-LD for a healthy subject the upper lung lobes may comprise from 55.08% up to 56.68%) of the total air volume, preferably comprise approximately 56%; whereas the lower lung lobes comprise from 43.50% up to 44.80% of the total air volume, preferably comprise approximately 44%. This specific distribution of airflow may be referred to as the FRC-LD upper vs lower lung lobes ratio, or simply the upper vs lower FRC-LD ratio. Accordingly, when referring to the "normal" upper vs lower FRC-LD ratio it may be understood that this refers to a range of (55.08-56.68):(43.50-44.80)%; preferably is a ratio approximately 56:44%.

Accordingly, when measuring the FRC-LD for a healthy subject the left lung lobes may comprise from 45.04% up to 46.35% of the total air volume, preferably comprise approximately 46%; whereas the right lung lobes comprise from 53.54% up to 55.14% of the total air volume, preferably comprise approximately 54%. This specific distribution of airflow may be referred to as the FRC-LD left vs right lung lobes ratio, or simply the left vs right FRC-LD ratio. Accordingly, when referring to the "normal" left vs right FRC-LD ratio it may be understood that this refers to a range of (45.04-46.35):(53.54-55.14)%; preferably is ratio of 46:54%.

In some embodiments the lobar distribution of the lungs may comprise (information as to) a volume of lower lung lobes and a volume of upper lung lobes. The upper vs lower lobes lobar distribution may serve as an indicator for the presence of a pulmonary condition.

In some further embodiments the system determines a TLC-LD of the upper vs lower lung lobes ratio. Preferably, the system determined upper vs lower lung lobes TLC-LD (%) may comprise at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 49:51 to 51:49; for example 50:50.

In some further embodiments the system determines a FRC-LD of the upper vs lower lung lobes ratio. Preferably, the system determined upper vs lower lung lobes FRC-LD (%) may comprise at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 57:43 to 55:45; for example 56:44.

In some embodiments the lobar distribution of the lungs may comprise (information as to) a volume of left lung lobes and a volume of right lung lobes. The left vs right lung lobes lobar distribution may also serve as an indicator for the presence of a pulmonary condition.

In some further embodiments the system determines a TLC-LD of left vs right lung lobes. Preferably, the system determined left vs right lung lobes TLC-LD (%) may comprise at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 45:55 to 47:53; for example 46:54.

In some further embodiments the system determines a FRC-LD of left vs right lung lobes. Preferably, the system determined left vs right lung lobes FRC-LD (%) may comprise at least 10:90 to at most 90:10; preferably 20:80 to 80:20; preferably 25:75 to 75:25; more preferably 30:70 to 70:30; more preferably 35:65 to 65:35; even more preferably 40:60 to 60:40; most preferably 45:55 to 47:53; for example 46:54.

In some further embodiments the lobar distribution of the lungs may comprise (information as to) a lobar distribution of lower lung lobes, a lobar distribution of upper lung lobes, a lobar distribution of left lung lobes and a lobar distribution of right lung lobes.

In some embodiments the lobar distribution of the lungs may comprise (information as to) a volume to each and every lung lobe. The each and every lung lobe comprise (information as to) the lobar distribution of the right upper lobe (RUL), the lobar distribution of the left upper lobe (LUL), the lobar distribution of the right middle lobe (RML), the lobar distribution of the right lower lobe (RLL) and the lobar distribution of the left lower lobe (LLL). Determining the volume of each lobe may provide for the highest accuracy and detect any compensation performed by any of the healthy or less affected lobe(s).

In some further embodiments the system determines a lobar distribution for each and every lung lobe for the TLC. Preferably, the system determined TLC-LD of the RUL lobe relative to the combined ratio of the other lung lobes (e.g. LUL, RML, RLL, and LLL) may comprise at least 0% to at most 40%; preferably at least 10% to at most 30%; more preferably 15% to at most 25%; most preferably 18% to 20%; for example 19%. Preferably, the system determined TLC-LD for the LUL lobe relative to the combined ratio of the other lung lobes (e.g. RUL, RML, RLL, and LLL) may comprise at least 0% to at most 40%; preferably at least 10% to at most 30%; more preferably 15% to at most 25%; most preferably 22 to 24%; for example 23%. Preferably, the system determined TLC-LD for the RML lobe relative to the combined ratio of the other lung lobes (e.g. RUL, LUL, RLL, and LLL) may comprise at least 0% to at most 20%; preferably at least 3% to at most 15%; more preferably 5% to at most 10%; most preferably 7% to 9%; for example 8%. Preferably, the system determined TLC-LD for the RLL lobe relative to the combined ratio of the other lung lobes (e.g. RUL, LUL, RML, and LLL) may comprise at least 0% to at most 40%; preferably at least 15% to at most 35%; more preferably 20% to at most 30%; most preferably 25% to 27%; for example 26%. Preferably, the system determined TLC-LD for the LLL lobe relative to the combined ratio of the other lung lobes (e.g. RUL, LUL, RML, and RLL) may comprise at least 0% to at most 40%; preferably at least 15% to at most 35%; more preferably 20% to at most 30%; most preferably 22% to 24%; for example 25%.

In some further embodiments the system determines a FRC-LD (FRC-LD) for each and every lung lobe. Preferably, the system determined FRC-LD of the RUL lobe relative to the combined ratio of the other lung lobes (e.g. LUL, RML, RLL, and LLL) may comprise at least 0% to at most 40%; preferably at least 10% to at most 30%; more preferably 15% to at most 25%; most preferably 20% to 22%; for example 21%. Preferably, the system determined FRC-LD for the LUL lobe relative to the combined ratio of the other lung lobes (e.g. RUL, RML, RLL, and LLL) may comprise at least 0% to at most 40%; preferably at least 10% to at most 30%; more preferably 15% to at most 25%; most preferably 24 to 26%; for example 25%. Preferably, system determined the FRC-LD for the RML lobe relative to the combined ratio of the other lung lobes (e.g. RUL, LUL, RLL, and LLL) may comprise at least 0% to at most 20%; preferably at least 3% to at most 15%; more preferably 5% to at most 10%; most preferably 9% to 11%; for example 10%. Preferably, the system determined FRC-LD for the RLL lobe relative to the combined ratio of the other lung lobes (e.g. RUL, LUL, RML, and LLL) may comprise at least 0% to at most 40%; preferably at least 15% to at most 35%; more preferably 20% to at most 30%; most preferably 22% to 24%; for example 23%. Preferably, the system determined FRC-LD for the LLL lobe relative to the combined ratio of the other lung lobes (e.g. RUL, LUL, RML, and RLL) may comprise at least 0% to at most 40%; preferably at least 15% to at most 35%; more preferably 20% to at most 30%; most preferably 20% to 22%; for example 21%.

The system may determine an internal airflow distribution (IAD), in particular to the lung lobes, in particular to the upper lung lobes and to the lower lung lobes, by querying a model or database created using a reference dataset of lADs and thoracic expansion patterns of healthy subjects and subjects with a pulmonary disorder.

A model or database may be queried with the registered sensor unit information and optionally presenting patient parameters. The model or database outputs the IAD, in particular lobar IAD, in particular IAD for the upper lung lobes and to the lower lung lobes.

A reference IAD for the database or model may calculated based on lobe volume values. A reference IAD may be determined from subject-specific medical images of the airways. To determine the IAD, medical images may be taken both at TLC (Total Lung Capacity) or full inhalation, and FRC (Functional Residual Capacity) or normal expiration. The IAD for a certain region in the lungs (lobes), is calculated as the ventilation going to that region (*i*.*e*. lobe volume at TLC minus lobe volume at FRC), divided by the total ventilation of the lungs (lung volume at TLC minus lobe volume at FRC). Therefore, IAD may be determined from the fraction of ventilation going to the area of interest (upper and lower lobes), with respect to the total ventilation of the lungs. The IAD may be calculated from medical images using the techniques described in Hajian B, De backer J, Vos W, Van holsbeke C, Clukers J, De backer W. Functional respiratory imaging (FRI) for optimizing therapy development and patient care. Expert Rev Respir Med. 2016;10(2): 193-206.

From the same medical images, reference exterior thoracic expansion patterns can be determined - these patterns can be detected by the sensor unit. The 3D models of the respective thoraxes recorded with medical imaging may be created and converted to a point cloud. The point cloud of the inspiratory medical images may be morphed onto a point cloud of expiratory medical images scans; this may be achieved, for instance, using a non-rigid coherent point drift (CPD) algorithm (A. Myronenko and X. Song, IEEE Transactions on Pattern Analysis and Machine Intelligence. Vol. 32, p. 2262 - 2275). The deformation of each point may be visualized using computer graphics and computer-aided design application software (Rhino 3D), focusing primarily on the distance between the original and the morphed point. Example 4 describes a process of determining exterior thoracic expansion patterns from medical images. As shown in Example 3, a pulmonary disorder affecting one or more lung zones or region results in changes to the local expansion of the thorax during a respiratory cycle (exhalation/inhalation). The database or model associates the changes to the local expansion of the thorax to IAD, which can be queried by the data from the sensor unit.

The sensor unit is not particularly limited to a specific technology and may suitably be selected by the skilled person from the commercially available technologies present or future so as to best fit the intended application thereof; in particular for registering the (local) expansion of the thorax or thoracic region of the subject. The terms thorax and thoracic region have been used interchangeable herein.

Generally speaking the sensor unit may be configured for measuring a mechanical deformation of the thorax of the subject, which reflects the local expansions of regions of the lung (e.g. lobes), and converting said deformation to a readable signal, such as an electric signal. The signal may be measurement data, or may be extrapolated to measurement data, optionally by comparison with an additional dataset(s) or user input. Typically the measurement data may be stored for future use, which may be locally stored on a storage medium, or be transferred to an external storage, such as a network comprising a server and database. Connected to or comprised within the sensor unit is preferably a device that manages and monitors all measurements performed by the sensor unit.

The data outputted by the sensor unit preferably corresponds to local upper and local lower thorax expansions.

The connection between the system and the sensor unit may be wired or wireless. Exemplary (wireless) communication types may without limitation be selected from the group consisting of Bluetooth (including, but not limited to Bluetooth Low Energy or Bluetooth Smart), LoRa, sub-GHz network (for example, SigFox, LoRa, LoRaWAN, ZigBee), wireless local area networking (WiFi), near-field communication (NFC), RFID chip, or antenna, hardware connections and cabling, and any combination of these.

The mechanical deformation can be measured in a number of ways; for example, piezoelectricity, changes of the electric resistance with the geometry, changes in electric capacity, acoustic changes in the resonant frequency of vibrating systems, and others. The sensor unit may comprise a combination of mechanical and electromechanical elements, which may be miniaturized (e.g. MEMS, NEMS). Additionally or alternatively, the mechanical deformation may also be recorded with an optical instrument(s) for recording or capturing images. The use of post-processing techniques may allow for accurately determining the expansions relative to a reference image.

Additionally, the sensor unit may comprise other components and features commonly used in the field of sensor technology; such as a power source or be connected to a power source, electronic circuitry, input device(s) for receiving information, output device for presentation of information in a visual or tactile form, transducers, actuators, and so on. Preferably the components of the sensor are partially or fully encased in a housing, which housing may be adapted for supporting and protecting said components.

The sensor unit comprises an optical sensor unit configured for registering a local expansion of the thorax or a thoracic region of the subject. In particular, the optical sensor unit may be configured for visually capturing the motion (*e*.*g*. expansion and contraction) of the thorax or a thoracic region.

An optical sensor unit may comprise an imaging sensor (*e*.*g*. CMOS, CCD). The optical sensor unit may comprise a lens system for focusing light from the object onto the imaging sensor. The optical sensor unit may comprise a scanner and/or a camera. The optical sensor unit is understood acquire images using light in the infrared (700 nm - 1000 nm), visible (400 nm - 700 nm), and/or ultraviolet (100 nm - 380 nm) spectrum, more preferably in a visible/IR light range (400 nm to 1000 nm). It captures images relating to the bodily exterior. The data outputted by the optical sensor unit preferably comprises local upper and local lower thorax expansions data.

The optical sensor unit may comprise a camera (*e*.*g*. 2D or 3D); light provided by a non-coherent light source may illuminate the object that is focused onto the imaging sensor. In particular for a camera, the camera preferably has parameters (*e*.*g*. resolution, aspect ratio, framerates, etc.) suitable for accurately distinguishing the degree of local expansion of the thorax. The camera may be configured for taking images at predetermined points, such as at rest and at maximal expansion, or may be configured for recording videos that allow post-processing.

The optical sensor unit may comprise a laser scanner; a light source such as a laser may be included within a housing of the optical sensor unit, or may be separate. The laser may projects a single or multiple stripes that may or may not cross, or form a geometric pattern.

The optical sensor may be configured for assisting in the calibration of the optical sensor with respect to the subject's position and/or thorax. This may be software assisted calibration, but may also comprise a dedicated device which provides feedback to the user, such as guidance lines. The optical sensor unit may be semi- or fully- automated, automatically capturing data when a suitable target is detected.

In some preferred embodiments the sensor unit may further comprise at least one mechanical sensor configured for skin-dismountable attachment to a thoracic region of the subject. The mechanical sensor may preferably be attached by means of suction or (temporary) adhesion, such as viscous substances which may or may not be activated by pressure, humidity, temperature, or other parameters. Alternatively, the sensor may be attached with an attachment means, such as a (self) adhesive tape, straps or pins. The sensor may also be part of a wearable device, which may be placed on or over the subject's thorax. Preferably the skin-dismountable attachment is painless and leaves no (permanent) markings on the skin of the subject.

In some embodiments the mechanical sensor is configured for detection of strain, linear acceleration, rotation, vibration, or sound. Accordingly, suitable sensors may include any one of a strain gauge, a piezo electric sensor, a force and torque sensor, an inertial sensor, a motion or speed sensor (e.g. accelerometer), a vibration sensor, a sound sensor (e.g. echo), and/or any combination thereof.

In some preferred embodiments the mechanical sensor is a strain gauge. The strain gauge measures the low-frequency volume changes associated with expansion and contraction of the chest during a normal breathing cycle. Also, low frequency and high-frequency information up to sound frequencies can be useful for screening breathing related parameters, such as patterns.

In some preferred embodiments the mechanical sensor is a piezoelectric sensor. The piezoelectric sensor measures the pressure, acceleration, strain, or force associated with expansion and contraction of the chest during a normal breathing cycle. Also, acceleration and force related information can be useful for screening breathing related parameters.

In some further embodiments the sensor unit further comprises at least two mechanical sensors (*e*.*g*. 2, 3, 4, 5, 6, 7, 8, 9 or more). The sensor unit may comprise two or more mechanical sensors of the same type (*e*.*g*. two or more strain gauges, two or more piezo electric, two or more accelerometers). The sensor unit may comprise two or more mechanical sensors of different types (*e*.*g*. two or more selected from strain gauge, piezo electric, or accelerometer). A plurality of mechanical sensors may allow for more accurately determining internal airflow distribution of the lungs.

In some further embodiments the sensor unit comprises at least two optical sensor units (*e*.*g*. 2, 3, 4, 5, 6, 7, 8, 9 or more). A plurality of optical sensor units may allow for more accurately determining internal airflow distribution of the lungs.

In some embodiments the sensor unit comprises at least one mechanical sensor, and at least one optical sensor unit. Combining different sensor types allows for verifying and comparing of information, which may improve the accuracy and reliability of the system.

In some embodiments each mechanical sensor is configured for attachment to a different position of the thoracic region of the subject. For instance, the at least two mechanical sensors are disposed on at least two (different) thoracic regions of a subject, thus being configured for determining two (different) local expansions of the thorax. The different regions may be related to anatomical regions, such as different ribs or lobe positions, or may be distance or surface area related, such as every n amount of cm (e.g. 5 cm, 10 cm, etc.).

In some embodiments the sensor unit comprises at least two mechanical sensors, which comprise a strain gauge and/or a piezo electric sensor. The local expansion of different regions of the thorax may be more accurately determined with different sensor types; for instance, the lower regions versus the upper regions.

Additionally or alternatively, each sensor is configured for attachment to a same position of the thoracic region of the subject. For instance, two different sensors may be placed on the same thoracic regions of a subject, thus allowing for a more accurate local measurement. They may be stacked, or be placed adjacent or contiguous to each other. The information may provide for comparative or supplementary data analysis, or may serve as a check-up to verify the integrity of each sensor measurement.

In some preferred embodiments the sensor unit comprises at least one mechanical sensor and at least one optical sensor unit. The combination of at least one mechanical and at least one optical sensor unit allows for a particularly accurate determination of the local expansions. Moreover, the locally attached mechanical sensor may serve as a reference point for determining the expansion with the optical sensor unit. For example, if the optical sensor comprises unit a laser, the laser may be configured to follow the displacement of a particular mechanical sensor. For example, if the optical sensor unit comprises a camera, the mechanical sensor captured on the image(s) of the optical sensor units may serve as a reference point during post-processing.

The sensor unit is configured for measurement of a local expansion of the thorax. The sensor unit is configured for measurement of expansion of the thorax at one or more local positions.

The local expansion of the thorax may be measured by direct placement of a mechanical sensor at the local position or positions to be measured. A mechanical sensors may be configured for attachment to the thorax of the subject at one local positions. Data outputted by the mechanical sensor placed at the local position indicates expansion of the thorax at the local position.

The local expansion of the thorax may be measured by determining from a series of optical images thorax movement at the local position or positions to be measured. The optical sensor unit may be configured for capture of optical images of the thorax wherein an image contains one or more local positions of the thorax. Data outputted by the optical sensor unit indicates expansion of the thorax at one or more local positions. Local expansion may be measured by tracking anatomical markers, placed along the contour lines representing maximal expansion in both lower and upper thorax (chest) areas. The local position or positions to be measured may be determined from a medical imaging study and correlation of medical images showing differential local thorax expansion with airflow distribution as explained elsewhere herein. The local positions may include the upper and lower thorax. A positional upper boundary to the upper thorax may be defined by the position of the 3rd intercostal space. A positional lower boundary to the lower thorax may be defined by the position of the xiphoid process. Registering the local expansion of the thorax of a subject implies also registration of a local contraction of the thorax. The sensor unit may be configured for registering a mechanical deformation of the thorax of the subject, which reflects the local expansions of regions of the lung (e.g. lobes), and converting said deformation to a readable signal, such as an electric signal.

In some embodiments the system is configured for determining a time-resolved internal airflow distribution of the lungs during a breathing cycle. A breathing cycle typically includes exhalation from total lung capacity (TLC) to functional residual capacity (FRC), and subsequent inhalation from functional residual capacity (FRC) to total lung capacity (TLC). Capturing breathing cycles may provide additional information about the pulmonary condition(s). For instance, different internal airflow rates may provide structural information about particular pulmonary obstructions and their location.

In some embodiments the system is configured for determining an integrated internal airflow distribution of the lungs during exhalation and during inhalation. The exhalation and inhalation speeds may be compared and/or superimposed to provide additional information about the pulmonary condition(s).

In some embodiments the system is further configured for determining the lower lobe volume and the upper lobe volume from a comparison between measurement data obtained during inhalation and measurement data obtained during exhalation.

In some embodiments the system is further configured for determining the lobar distribution from a comparison between measurement data obtained during inhalation and measurement data obtained during exhalation.

In some preferred embodiments the system is further configured for determining the volume each and every lung lobe from a comparison between measurement data obtained during inhalation and measurement data obtained during exhalation.

In some embodiments the system is further configured to determine, from the internal airflow distribution of the lungs of the subject, a status of the subject with respect to a pulmonary disorder. As described above, the status of the subject may be considered an indicator for determining whether a subject is at risk for being affected by a pulmonary condition. The status may be a Boolean value, such as "at risk" or "not at risk", but may also be a percentage based value, such as 90% of risk, or 50% of risk, or any other numerical value suitable for indicating the risk for a pulmonary condition.

Moreover, the status may also be accorded a specific pulmonary disorder type, such at risk for IPF, or at risk for ILD.

In some embodiments the pulmonary disorder is IPF, Asthma, COPD, CF, BOS, non-CF bronchiectasis, PH, BPD, A1AT, ILD, or any combination thereof. In principle, any pulmonary disorder that may structurally affect the lungs causing a deviation of the internal airflow distribution from the observed norm for a healthy subject may be considered as determinable with the present system.

In some further embodiments the system comprises an interface for reporting the internal airflow distribution parameters determined internal airflow in the lungs to a healthcare professional. The interface may be provided through an output device, which may report the parameters in a visual or tactile form.

In some embodiments, the interface is configured for indicating whether the presenting subject's internal airflow distribution is within or outside the normal range. The ranges may be predefined, but may preferably be adapted based on subject information parameters, which may include subject gender, physical fitness, health, etc. The presenting subject parameters may be entered by a user of the system, for instance through an input device, but may also be received from an external device, such as a network comprising (a plurality of) subject information.

In some embodiments, the interface is configured to suggest follow-up functional respiratory imaging measurements when the internal airflow distribution is outside the normal range. The suggestion may be based on the above described status of the subject.

A further aspect of the invention provides a computer-implemented method for airflow determining distribution in lungs of a subject comprising the steps:
- receiving sensor data from the sensor unit configured for registering a local expansion of the thorax or a thoracic region of the subject;
- computing, from the sensor unit data, local expansion of the thorax and spatially and/or temporally resolved volume changes in the subject's lungs;
- computing, from the volume changes, airflow distribution in the subject's lungs; wherein the airflow distribution is optionally temporally resolved.

The sensor unit comprises at least one optical sensor unit.

In some embodiments the method further comprises the step of:
- computing from the airflow distribution in the subject's lungs, the IAD upper vs lower lobes ratio, the IAD left vs right lobes ratio, and/or the internal airflow to each and every lung lobe (ratio).

In some embodiments the method further comprises the step of:
- determining from the airflow distribution in the subject's lungs, a status of the subject with respect to a pulmonary disorder.

In some embodiments the method is executed by means of a system according to one or more embodiments as described herein.

A further aspect of the invention provides a use of a system according to one or more embodiments as described herein.

In some embodiments the use of the system is for determining a status of the subject with respect to a pulmonary disorder. In some embodiments the pulmonary disorder is IPF, Asthma, COPD, CF, BOS, non-CF bronchiectasis, PH, BPD, A1AT, ILD, or any combination thereof. In some embodiments the use of the system is for determining a status of the subject with respect to IPF.

In some embodiments the use of the system is for determining a fitness of a subject. Preferably, the fitness comprises an exercise tolerance of the subject. For instance, the fitness may be an indicator of the physical fitness of the subject, which may be a sportsman or athlete, which fitness may be useful to follow-up on training regimes, biological capabilities, diets, and/or other exercise related parameters.

In some embodiments the use of the system is for determining a recovery of a subject. Preferably, the recovery is a local recovery of a lung lobe and/or a lung region. For instance, after a treatment or surgery, preferably long-related surgery, the recovery may be an indicator of the subject's response to the treatment or surgery.

### EXAMPLES

### Example 1

Reference is made to **Table 1**. **Table 1** presents an overview of the standard IAD range observed for a healthy subject (i.e. a subject without pulmonary conditions) for each and every lung lobe and for specific lung zones.

**Table 1: Standard IAD values for a healthy subject; wherein estimate = the mean value predicted by the mixed-effect model and CL = 95% confidence level.**

| **Lung zone.** | **Estimate** | **Lower CL** | **Upper CL** |
|---|---|---|---|
| RUL | 16.61 | 16.05 | 17.19 |
| RML | 6.28 | 6.02 | 6.55 |
| RLL | 28.72 | 27.85 | 29.61 |
| LUL | 20.79 | 20.11 | 21.48 |
| LLL | 26.55 | 25.73 | 27.39 |
| Upper zone (RLL, LUL, RML) | 43.68 | 42.75 | 44.61 |
| Lower zone (RLL, LLL) | 55.27 | 54.06 | 56.48 |
| Left zone (LUL, LLL) | 47.34 | 46.26 | 48.41 |
| Right zone (RUL, RML, RLL) | 51.61 | 50.53 | 52.70 |

The data presented in Table 1 was based on a mixed-effect model with the fixed effects: lobe + sex * height + age, and the random intercept: subject. After reduction of the model, the IAD was observed to be only dependent on the lobe or lung zone.

In an exemplary embodiment, the presented Table 1 may serve as a reference when determining a status of a subject with respect to a pulmonary disorder.

In particular, when an IAD in a first subject's lungs is observed as falling within the standard IAD range presented in Table 1, for instance an IAD upper vs lower lung lobes ratio of 44:46 (%), the first subject may be assigned a negative status, which may be indicative of a lower risk of a potential pulmonary disorder.

However, when an IAD in a second subject's lungs is observed as falling outside of the standard IAD range presented in Table 1, for instance an IAD upper vs lower lung lobes ratio of 50:50 (%), the second subject may be assigned a positive status, which may be indicative of a higher risk of a potential pulmonary disorder. Moreover, the abnormal ratio may for instance serve as an indication that the upper lungs are partially compensating for the lower lungs.

For comparative purposes, the standard lobar distribution is provided for a healthy subject with reference to **Tables 2** and **3. Table 2** presents an overview of the standard lobar distribution range observed when measuring the total lung capacity (TLC) of a healthy subject for each and every lung lobe and for specific lung zones; whereas **Table 3** presents an overview of the standard values when measuring the functional residual capacity (FRC).

**Table 2: Standard Lobar distribution at TLC values for a healthy subject; wherein estimate = the mean value predicted by the mixed-effect model and CL = 95% Confidence level.**

| **Lung zone.** | **Estimate** | **Lower CL** | **Upper CL** |
|---|---|---|---|
| RUL | 18.77 | 18.29 | 19.25 |
| RML | 8.21 | 7.97 | 8.46 |
| RLL | 25.91 | 25.29 | 26.53 |
| LUL | 23.02 | 22.46 | 23.59 |
| LLL | 23.48 | 22.91 | 24.06 |
| Upper (RLL, LUL, RML) | 50.00 | 49.22 | 50.78 |
| Lower (RLL, LLL) | 49.39 | 48.54 | 50.23 |
| Left (LUL, LLL) | 46.50 | 45.69 | 47.30 |
| Right (RUL, RML, RLL) | 52.89 | 52.06 | 53.71 |

The data presented in Table 2 are based on a mixed-effect model with the fixed effects: lobe + sex * height + age, and the random intercept: subject. After reduction of the model, the lobar distribution at TLC was observed to be only dependent on the lobe or lung zone.

**Table 3: Lobar distribution at FRC values for a healthy subject; wherein estimate = the mean value predicted by the mixed-effect model and CL = 95% Confidence level.**

| **Lung zone.** | **Estimate** | **Lower CL** | **Upper CL** |
|---|---|---|---|
| RUL | 20.72 | 20.26 | 21.18 |
| RML | 10.15 | 9.69 | 10.61 |
| RLL | 23.46 | 23.00 | 23.92 |
| LUL | 25.01 | 24.55 | 25.47 |
| LLL | 20.69 | 20.23 | 21.15 |
| Upper (RLL, LUL, RML) | 55.88 | 55.08 | 56.68 |
| Lower (RLL, LLL) | 44.15 | 43.50 | 44.80 |
| Left (LUL, LLL) | 45.70 | 45.04 | 46.35 |
| Right (RUL, RML, RLL) | 54.34 | 53.54 | 55.14 |

The data presented in Table 3 are based on a mixed-effect model with the fixed effects: lobe + sex * height + age, and the random intercept: subject. After reduction of the model, the lobar distribution at FRC was observed to be only dependent on the lobe or lung zone.

In an exemplary embodiment, the presented Table 2 may serve as a reference when determining a status of the subject with respect to a pulmonary disorder. Similar observations hold true for Table 3.

In particular, when a measured lobar distribution in a first subject's lungs is observed as falling within the standard lobar distribution range presented in Table 2, for instance an upper vs lower lung lobes lobar distribution ratio of 50:50 (%), the first subject may be assigned a negative status, which may be indicative of a lower risk of a potential pulmonary disorder.

However, when a measured lobar distribution in a second subject's lungs is observed as falling outside of the standard lobar distribution range presented in Table 2, for instance an upper vs lower lung lobes lobar distribution ratio of 55:45 (%), the second subject may be assigned a positive status, which may be indicative of a higher risk of a potential pulmonary disorder. Moreover, the abnormal ratio may for instance serve as an indication that the upper lungs are partially compensating for the lower lungs.

### Example 2

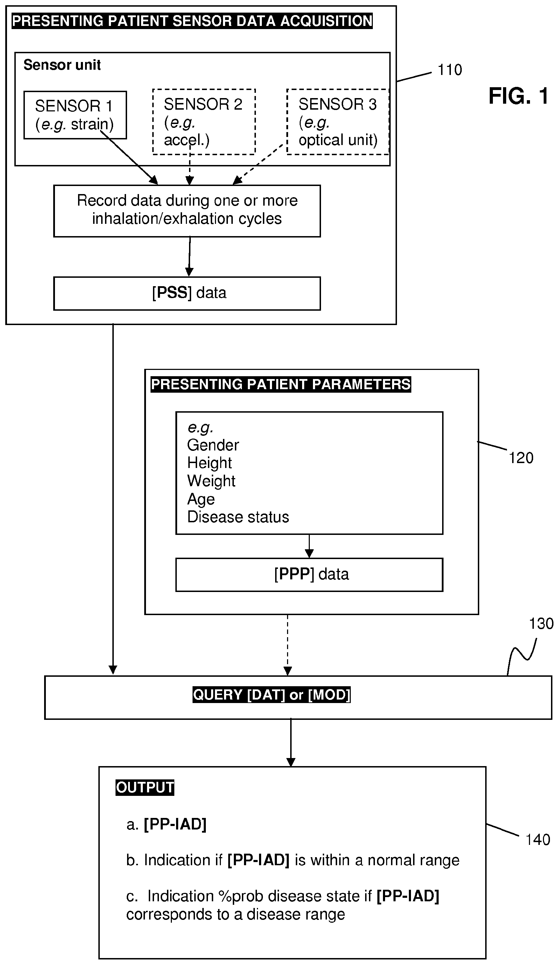

Reference is made to **FIG. 1. FIG. 1** shows a flow-chart for performing the method according to one or more embodiments as described herein.

In this exemplary embodiment, sensor data [PSS] is obtained of a presenting subject or patient through data acquisition (110). The [PSS] data may be obtained from a sensor unit as described herein comprising multiple mechanical sensors, such as a first mechanical sensor that is a strain sensor, a second mechanical sensor that is an accelerometer. The sensor unit may comprise a third sensor that is an optical sensor unit. The [PSS] data may be obtained during one or more inhalation/exhalation cycles, which may allow for computing spatially and/or temporally resolved volume changes. In addition, presenting patient parameters [PPP] may be noted (120) such as gender, height, weight, age, disease status.

The [PPS] data and optionally the [PPP] are queried (130) against a model [MOD] or database [DAT]. Outputted (140) may be one or more of
(a) a presenting patient internal airflow distribution [PP-IAD],
(b) an indication if [PP-IAD] is within a normal range,
(c) an indication of a probability of a condition if [PP-IAD] corresponds to a disease range,

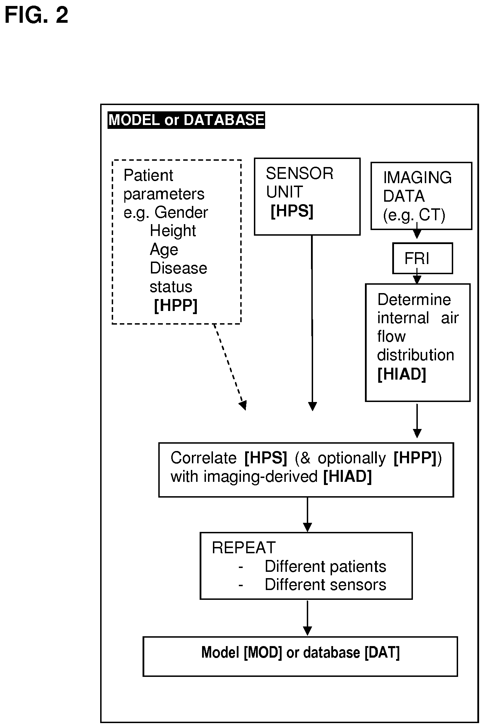

**FIG. 2** is an example of how the reference database [DAT] or model [MOD] is formed. For each of a plurality of historic patient cases, historic patient sensor unit data [HPS], optionally historic patient parameters [HPP] and historic internal airflow distribution [HIAD] of the historic patient. [HIAD] is determined by functional respiratory imaging (FRI) of imaging data (CT or MR). By historic it is meant that the data is obtained previously from the subject. From the data a model [MOD] or database [DAT] may be created that correlates [HS] and optionally [HPP] with imaging-derived [lAD]. To improve the model or database, data from multiple historic patients is entered.

Standard or predetermined ranges for air flow distribution which may indicate that a degree of health of a subject. Standard or predetermined ranges for air flow distribution which may indicate that a subject is affected by a pulmonary condition. Preferably the latter may allow assigning a status to the subject with respect to a pulmonary disorder.

### Example 3

To identify the thoracic regions which are most indicative of a pulmonary disorder a series of HRCT medical imaging scans were performed. For a total of 30 subjects lying in supine position the local thoracic expansions were recorded. From the medical imaging scans the subject's skin was segmented away from the remaining data. The scanned skin could then be reconstructed as 3D models representative of the average expiratory and inspiratory levels. From these reconstructions an accurate assessment could be made of the skin displacement across the entire thorax for every subject.

From the 30 subjects 15 were known to be healthy (i.e. lacking any pulmonary disorder) and served as reference, while 15 were known to be affected with the pulmonary disorder IPF and were expected to show an abnormal airflow as a result.

The 3D models of the respective thoraxes recorded with HRCT imaging were converted to a point cloud. The amount of data points was kept below 5000 to limit running time; however, this number could be increased if necessary and/or with suitable computing hardware. The point cloud of the inspiratory medical imaging scans was morphed onto the point cloud of the medical imaging expiratory scans using a non-rigid coherent point drift (CPD) algorithm (reference: A. Myronenko and X. Song, IEEE Transactions on Pattern Analysis and Machine Intelligence. Vol. 32, p. 2262 - 2275). The deformation of each point was visualized using a computer graphics and computer-aided design application software (Rhino 3D), focusing primarily on the distance between the original and the morphed point.

Reference is made to **FIGs. 3A** and **B**. An average image was made by overlaying the results of every subject for each of the each of the two groups. In particular, **FIG. 3A** shows the (overlayed) thoracic expansion computed from the subject's sensor data received from the healthy subjects; whereas **FIG. 3B** shows the (overlayed) thoracic expansion from the subjects affected with the pulmonary disorder IPF.

To summarize the results: **FIG. 3A** illustrates a normal thoracic expansion pattern wherein the entire thorax is observed to expand, with the upper thoracic region expanding slightly more than the middle and lower region. Accordingly, for a subject in supine position thorax primarily expands centrally upwards.

By comparison, **FIG. 3B** illustrates a thoracic expansion pattern that is affected by a pulmonary disorder. Contrary to **FIG. 3A****,** almost no expansion of the middle and lower thoracic region can be observed. Instead the upper thoracic region is observed to expand far more intensely and laterally, extending along the subject's sides in supine position. These observations may indicate that the upper lung lobes are compensating for the lower lung lobes, which is in line with the expectations for lungs affected with the pulmonary disorder IPF.

In view of the above, it is confirmed that a clear distinction can be made between thoracic expansion patterns for healthy and non-healthy subjects. These local skin expansions are registerable using a sensor unit, in particular when focusing on the affected and compensating lung regions.

### Example 4

Medical images of subjects at Functional Residual Capacity (FRC), or normal exhalation and Total Lung Capacity (TLC), or full inhalation, are gathered for both cohorts, IPF and healthy. From those medical images, the thorax is segmented for each subject. The region of interest for the full thorax region is determined by the first vertebra just above the lungs (upper boundary) and the first vertebra just under the lungs (lower boundary).

The segmented chest models are converted to point clouds. These comprise grids with a fixed amount of grid points over all patients and all medical images. The grid covers the entire chest model.

By means of non-rigid coherent point drift algorithms, grid points on FRC levels are mapped with grid points on TLC level per patient. This process results in a transformation matrix, allowing for a calculation of absolute displacement vectors from the FRC grid points to the corresponding TLC grid points.

The thorax is divided into upper and lower regions, corresponding to upper and lower lobes, by means of patient-specific anatomical landmarks, including the xiphoid process and the 3rd intercostal space (based on literature, Bockenhauer SE, Chen H, Julliard KN, Weedon J. Measuring thoracic excursion: reliability of the cloth tape measure technique. J Am Osteopath Assoc. 2007;107(5):191-6). A dividing line between upper and lower thorax may be determined by the xiphoid process and 3rd intercostal space as outer boundaries and is placed from a fixed distance (optimal distance to be determined, see below) from the top vertebra (top of segmented chest).

The ratio of total upper and lower thorax expansions is calculated, cancelling out patient-specific factors (mostly patient size). The ratios are calculated for both the healthy population as for the IPF population for different levels of the dividing line (going from xiphoid process to 3rd intercostal space). The dividing line yielding the biggest difference in chest expansion ratio for the healthy and IPF cohort is the ideal dividing line.

Once this division is obtained, confined upper and lower thorax zones for contour lines are identified that experience the greatest elongation going from FRC (exhalation) to TLC (normal inhalation), for both the healthy and IPF patients. These contour lines determine the eventual positioning of locally-positioned mechanical sensors and/or markers for the optical sensors, representing expansion in upper and lower thorax in the most generic way.

### Example 5

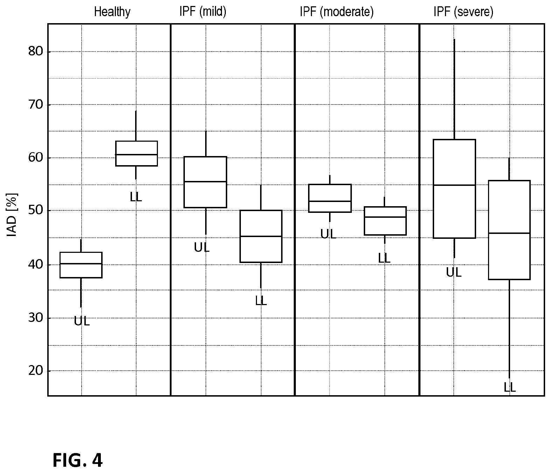

IAD between upper and lower lung lobes was measured in healthy subjects and in subjects with idiopathic pulmonary fibrosis (IPF) at different disease stages (mild, moderate, severe) based medical images of the subjects at Functional Residual Capacity (FRC) and Total Lung Capacity (TLC). In total 30 healthy patients, 4 mild, 14 moderate and 24 severe IPF patients were analyzed. The IAD for each of the upper and lower lobes was calculated as the ventilation going to each lobe *i.e.* lobe volume at TLC minus lobe volume at FRC, divided by the total ventilation of the lungs *i.e.* lung volume at TLC minus lobe volume at FRC. In healthy subjects 40% of the inspired air was directed towards the upper lobes (UL) and 60% of the air was directed towards the lower lobes (LL). In subjects with IPF this ratio was consistently altered in all disease stages (mild to severe) as shown in **FIG. 4**. The results show that changes in IAD are caused by the heterogeneous nature of IPF disease manifestations with greater and earlier impact on the lower lobes.

## Claims

1. A system comprising a sensor unit configured for registering an expansion of a thorax of a subject, the system being configured for determining from data outputted by the sensor unit a local expansion of the thorax and an internal airflow distribution, IAD, of the lungs of the subject, wherein
- the sensor unit sensor unit comprises an optical sensor unit,
- the IAD of the lungs of the subject comprises an IAD to lower lung lobes and an IAD to upper lung lobes; and,
the at least one optical sensor unit comprises a scanner and/or a camera, configured to capture optical images of the upper thorax and the lower thorax of the subject.

2. The system according to claim 1, wherein the sensor unit further comprises at least two mechanical sensors configured for skin-dismountable attachment to a thoracic region of the subject.

3. The system according to claim 1 or 2, wherein the data outputted by the sensor unit comprises data corresponding to local upper and local lower thorax expansions of the subject.

4. The system according to any one of claims 1 to 3, wherein the system determines an IAD to the upper lung lobes and IAD to the lower lung lobes by querying a model or database created using a reference dataset of lobar upper and lower lADs and thoracic expansion patterns of healthy subjects and subjects with a pulmonary disorder;

5. The system according to claim 4, wherein the reference dataset of lADs and thoracic expansion patterns are determined from medical images obtained by medical imaging of healthy subjects and subjects with a pulmonary disorder at total lung capacity and functional residual capacity.

6. The system according to any one of claims 1 to 5, wherein data outputted by the sensor unit corresponds to local upper and local lower thorax expansions of the subject, wherein a positional upper boundary to the upper thorax is defined by the 3rd intercostal space, and a positional lower boundary to the lower thorax is defined by the xiphoid process.

7. The system according to any one of claims 1 to 6, wherein the sensor unit comprises at least two mechanical sensors one configured for attachment to the upper thorax and one configured for attachment to the lower thorax of the subject.

8. The system according to any one of claims 1 to 7, wherein the mechanical sensor is a strain gauge or a piezo electric sensor, optionally wherein the sensor unit comprises a strain gauge and piezo electric sensor.

9. The system according to any one of claims 1 to 8, wherein the system is configured for determining a time-resolved internal airflow distribution of the lungs during a breathing cycle.

10. The system according to any one of claims 1 to 9, wherein the system is configured for determining an integrated internal airflow distribution of the lungs during exhalation and during inhalation.

11. The system according to any one of claims 1 to 10, wherein the system is further configured for determining the lower lobe volume and the upper lobe volume from a comparison between measurement data obtained during inhalation and measurement data obtained during exhalation.

12. The system according to any one of claims 1 to 11 further configured to determine, from the internal airflow distribution of the lungs of the subject, a status of the subject with respect to a pulmonary disorder.

13. The system according to claim 12, wherein the pulmonary disorder is idiopathic pulmonary fibrosis, IPF, Asthma, COPD, CF, BOS, non-CF bronchiectasis, PH, BPD, A1AT, ILD, or any combination thereof, preferably idiopathic pulmonary fibrosis.

14. The system according to claim 12, wherein the system is configured to identify a normal status of subject when the IAD to the upper lung lobes is 40 to 45%, and when the IAD to the lower lung lobes is 55 to 60%.

15. Computer-implemented method for determining internal airflow distribution, IAD, in lungs of a subject comprising the steps:
- receiving sensor data from a sensor unit comprising at least an optical sensor unit comprising a scanner and/or a camera, the sensor unit being configured for registering a local expansion of the thorax or a thoracic region of the subject, the sensor data corresponding to local upper and local lower thorax expansions of the subject;
- computing, from the sensor unit data, local expansion of the thorax and spatially and/or temporally resolved volume changes in the subject's lungs; and
- computing, from the volume changes, the IAD in the subject's lower lung lobes and upper lung lobes; wherein the airflow distribution is optionally temporally resolved.

## Patentansprüche

1. System, das eine Sensoreinheit umfasst, die dafür ausgelegt ist, eine Ausdehnung eines Brustkorbs eines Probanden zu registrieren, wobei das System dafür ausgelegt ist, aus den von der Sensoreinheit ausgegebenen Daten eine lokale Ausdehnung des Brustkorbs und eine interne Luftstromverteilung, IAD [Internal Airflow Distribution], der Lungen des Probanden zu bestimmen, wobei
- die Sensoreinheit eine optische Sensoreinheit umfasst,
- die IAD der Lungen des Probanden eine lAD zu den unteren Lungenflügeln und eine lAD zu den oberen Lungenflügeln umfasst; und
die wenigstens eine optische Sensoreinheit einen Scanner und/oder eine Kamera umfasst, die dafür ausgelegt ist/sind, optische Bilder des oberen Brustkorbs und des unteren Brustkorbs des Probanden zu erfassen.

2. System gemäß Anspruch 1, wobei die Sensoreinheit ferner wenigstens zwei mechanische Sensoren umfasst, die für eine von haut abnehmbare Befestigung an einem Brustkorbbereich des Probanden ausgelegt sind.

3. System gemäß Anspruch 1 oder 2, wobei die von der Sensoreinheit ausgegebenen Daten Daten umfassen, die den lokalen Ausdehnungen des oberen und des unteren Brustkorbs des Probanden entsprechen.

4. System gemäß einem der Ansprüche 1 bis 3, wobei das System eine lAD zu den oberen Lungenflügeln und eine lAD zu den unteren Lungenflügeln bestimmt, indem es ein Modell oder eine Datenbank abfragt, das bzw. die unter Verwendung eines Referenzdatensatzes von IADs der oberen und unteren Lungenflügel und Brustkorbausdehnungsmustern von gesunden Probanden und Probanden mit einer Lungenerkrankung erstellt wurde.

5. System gemäß Anspruch 4, wobei der Referenzdatensatz von IADs und Brustkorbausdehnungsmustern aus medizinischen Bildern bestimmt wird, die durch medizinische Bildgebung von gesunden Probanden und Probanden mit einer Lungenerkrankung bei Gesamtlungenkapazität und funktionaler Restkapazität gewonnen werden.

6. System gemäß einem der Ansprüche 1 bis 5, wobei die von der Sensoreinheit ausgegebenen Daten lokalen Ausdehnungen des oberen und des unteren Brustkorbs des Probanden entsprechen, wobei eine obere Positionsgrenze zum oberen Brustkorb durch den dritten Interkostalraum und eine untere Positionsgrenze zum unteren Brustkorb durch den Xiphoidfortsatz definiert ist.

7. System gemäß einem der Ansprüche 1 bis 6, wobei die Sensoreinheit wenigstens zwei mechanische Sensoren umfasst, von denen einer für die Befestigung am oberen Brustkorb und einer für die Befestigung am unteren Brustkorb des Probanden ausgelegt ist.

8. System gemäß einem der Ansprüche 1 bis 7, wobei der mechanische Sensor ein Dehnungsmessstreifen oder ein piezoelektrischer Sensor ist, wobei die Sensoreinheit optional einen Dehnungsmessstreifen und einen piezoelektrischen Sensor umfasst.

9. System gemäß einem der Ansprüche 1 bis 8, wobei das System dafür ausgelegt ist, eine zeitaufgelöste interne Luftstromverteilung der Lunge während eines Atemzyklus zu bestimmen.

10. System gemäß einem der Ansprüche 1 bis 9, wobei das System dafür ausgelegt ist, eine integrierte interne Luftstromverteilung der Lunge während der Ausatmung und während der Einatmung zu bestimmen.

11. System gemäß einem der Ansprüche 1 bis 10, wobei das System ferner dafür ausgelegt ist, das Volumen der unteren Lungenflügel und das Volumen der oberen Lungenflügel anhand eines Vergleichs zwischen während der Einatmung erhaltenen Messdaten und während der Ausatmung erhaltenen Messdaten zu bestimmen.

12. System gemäß einem der Ansprüche 1 bis 11, das ferner dafür ausgelegt ist, anhand der internen Luftstromverteilung der Lunge des Probanden einen Status des Probanden in Bezug auf eine Lungenerkrankung zu bestimmen.

13. System gemäß Anspruch 12, wobei die Lungenerkrankung idiopathische Lungenfibrose, IPF, Asthma, COPD, CF, BOS, Non-CF-Bronchiektase, PH, BPD, A1AT, ILD oder eine beliebige Kombination davon ist, vorzugsweise idiopathische Lungenfibrose.

14. System gemäß Anspruch 12, wobei das System dafür ausgelegt ist, einen normalen Status des Probanden zu identifizieren, wenn die IAD zu den oberen Lungenflügeln 40 bis 45 % beträgt und wenn die IAD zu den unteren Lungenflügeln 55 bis 60 % beträgt.

15. Computerimplementiertes Verfahren zum Bestimmen der internen Luftstromverteilung, IAD [Internal Airflow Distribution], in der Lunge eines Probanden, das die Schritte umfasst:
- Empfangen von Sensordaten von einer Sensoreinheit, die wenigstens eine optische Sensoreinheit mit einem Scanner und/oder einer Kamera umfasst, wobei die Sensoreinheit dafür ausgelegt ist, eine lokale Ausdehnung des Brustkorbs oder eines Brustkorbbereichs des Probanden zu registrieren, wobei die Sensordaten lokalen Ausdehnungen des oberen und des unteren Brustkorbs des Probanden entsprechen;
- Berechnen, anhand der Daten der Sensoreinheit, der lokalen Ausdehnung des Brustkorbs und der räumlich und/oder zeitlich aufgelösten Volumenänderungen in der Lunge des Probanden; und
- Berechnen der IAD in den unteren Lungenflügeln und den oberen Lungenflügeln des Probanden aus den Volumenänderungen; wobei die Luftstromverteilung optional zeitlich aufgelöst wird.

## Revendications

1. Système comprenant une unité de capteur configurée pour enregistrer une expansion du thorax d'un sujet, le système étant configuré pour déterminer, à partir des données fournies par l'unité de capteur, une expansion locale du thorax et une distribution du flux d'air interne, IAD [Internal Airflow Distribution], des poumons du sujet, dans lequel :
- l'unité de capteur comprend une unité de capteur optique,
- l'IAD des poumons du sujet comprend une IAD pour les lobes pulmonaires inférieurs et une IAD pour les lobes pulmonaires supérieurs ; et
l'au moins une unité de capteur optique comprend un scanner et/ou une caméra, configurés pour capturer des images optiques de la partie supérieure du thorax et de la partie inférieure du thorax du sujet.

2. Système selon la revendication 1, dans lequel l'unité de capteur comprend en outre au moins deux capteurs mécaniques configurés pour une fixation démontable sur la peau d'une région thoracique du sujet.

3. Système selon la revendication 1 ou la revendication 2, dans lequel les données émises par l'unité de capteur comprennent des données correspondant à des expansions locales supérieures et inférieures du thorax du sujet.

4. Système selon l'une quelconque des revendications 1 à 3, dans lequel le système détermine une IAD pour les lobes pulmonaires supérieurs et une IAD pour les lobes pulmonaires inférieurs en interrogeant un modèle ou une base de données créé à l'aide d'un ensemble de données de référence d'IAD de lobes supérieurs et inférieurs et de schémas d'expansion thoracique de sujets sains et de sujets souffrant d'un trouble pulmonaire.

5. Système selon la revendication 4, dans lequel l'ensemble de données de référence des IAD et des schémas d'expansion thoracique est déterminé à partir d'images médicales obtenues par imagerie médicale de sujets sains et de sujets atteints d'un trouble pulmonaire à la capacité pulmonaire totale et à la capacité pulmonaire résiduelle fonctionnelle.

6. Système selon l'une quelconque des revendications 1 à 5, dans lequel les données émises par l'unité de capteur correspondent à des expansions thoraciques supérieures et inférieures locales du sujet, une limite supérieure positionnelle pour le thorax supérieur étant définie par le troisième espace intercostal et une limite inférieure positionnelle pour le thorax inférieur étant définie par le processus xiphoïde.

7. Système selon l'une quelconque des revendications 1 à 6, dans lequel l'unité de capteur comprend au moins deux capteurs mécaniques, l'un étant configuré pour être fixé à la partie supérieure du thorax et l'autre étant configuré pour être fixé à la partie inférieure du thorax du sujet.

8. Système selon l'une quelconque des revendications 1 à 7, dans lequel le capteur mécanique est une jauge de contrainte ou un capteur piézoélectrique, dans lequel, éventuellement, l'unité de capteur comprend une jauge de contrainte et un capteur piézoélectrique.

9. Système selon l'une quelconque des revendications 1 à 8, dans lequel le système est configuré pour déterminer une distribution de flux d'air interne des poumons résolue dans le temps au cours d'un cycle respiratoire.

10. Système selon l'une quelconque des revendications 1 à 9, dans lequel le système est configuré pour déterminer une distribution de flux d'air interne intégrée des poumons pendant l'expiration et pendant l'inspiration.

11. Système selon l'une quelconque des revendications 1 à 10, dans lequel le système est en outre configuré pour déterminer le volume du lobe inférieur et le volume du lobe supérieur à partir d'une comparaison entre les données de mesure obtenues pendant l'inspiration et les données de mesure obtenues pendant l'expiration.

12. Système selon l'une quelconque des revendications 1 à 11, en outre configuré pour déterminer, à partir de la distribution du flux d'air interne des poumons du sujet, un état du sujet par rapport à un trouble pulmonaire.

13. Système selon la revendication 12, dans lequel le trouble pulmonaire est une fibrose pulmonaire idiopathique, FPI, l'asthme, la COPD, la CF, la BOS, la bronchectasie non CF, la PH, la DBP, l'A1AT, l'ILD, ou toute combinaison de ces affections, de préférence la fibrose pulmonaire idiopathique.

14. Système selon la revendication 12, dans lequel le système est configuré pour identifier un état normal du sujet lorsque l'IAD pour les lobes pulmonaires supérieurs est de 40 à 45 %, et lorsque l'IAD pour les lobes pulmonaires inférieurs est de 55 à 60 %.

15. Procédé mis en oeuvre par ordinateur pour déterminer la distribution du flux d'air interne, IAD [Internal Airflow Distribution], dans les poumons d'un sujet, le procédé comprenant les étapes suivantes :
- recevoir des données de capteur provenant d'une unité de capteur comprenant au moins une unité de capteur optique comprenant un scanner et/ou une caméra, l'unité de capteur étant configurée pour enregistrer une expansion locale du thorax ou d'une région thoracique du sujet, les données de capteur correspondant à des expansions locales supérieures et inférieures du thorax du sujet ;
- calculer, à partir des données de l'unité de capteur, l'expansion locale du thorax et les changements de volume résolus spatialement et/ou temporellement dans les poumons du sujet ; et
- calculer, à partir des changements de volume, l'IAD dans les lobes pulmonaires inférieurs et les lobes pulmonaires supérieurs du sujet ; la distribution du flux d'air étant éventuellement résolue dans le temps.
